# FASCICULE DE BREVET EUROPEEN

(11) **EP 1 385 886 B1**
(45) Date de publication et mention de la délivrance du brevet: **07.12.2005**
(21) Numéro de dépôt: 02727688.0
(22) Date de dépôt: 24.04.2002
(51) Int. Cl.: C07K 16/06, C07K 1/18, C07K 1/36, C07K 1/30

(54) **PROCEDE DE PREPARATION DE CONCENTRES D'IMMUNOGLOBULINES HUMAINES A USAGE THERAPEUTIQUE**
VERFAHREN ZUR HERSTELLUNG VON KONZENTRIERTEN MENSCHLICHEN IMMUNOGLOBULINEN FÜR DIE THERAPEUTISCHE VERWENDUNG
METHOD FOR PREPARING HUMAN IMMUNOGLOBULIN CONCENTRATES FOR THERAPEUTIC USE

(30) Priorité: 11.05.2001 FR 0106234
(43) Date de publication de la demande: 04.02.2004
(73) Titulaire: Laboratoire Français du Fractionnement et des Biotechnologies, 91940 Les Ulis (FR)
(72) Inventeur: CHTOUROU, Abdessatar, Sami, F-78990 Elancourt (FR); PAOLANTONACCI, Philippe, F-91190 Gif sur Yvette (FR); SCHMITTHAEUSLER, Roland, F-78180 Montigny le Bretonneux (FR); LIROCHON, Jacky, F-91650 Breuillet (FR)
(74) Mandataire: Lepeudry-Gautherat, Thérèse
(86) Numéro de dépôt international: PCT/FR2002/001407
(87) Numéro de publication internationale: WO 2002/092632

(56) Documents cités:
- US-A- 4 305 870
- US-A- 5 075 425
- US-A- 5 808 000
- US-A- 6 069 236

## Description

L'invention concerne un procédé de préparation de concentrés d'immunoglobulines humaines à usage thérapeutique, à partir de plasma ou d'une fraction de plasma humain. Le procédé permet d'obtenir des concentrés d'immunoglobulines G (IgG), d'immunoglobulines A (IgA) et d'immunoglobulines M (IgM).

L'utilisation de fractions de plasma humain enrichies en immunoglobulines pour le traitement de diverses infections ou déficiences congénitales est connue depuis la mise au point du procédé de précipitation à l'éthanol par Cohn (Cohn et al. 1946, J. Am. Chem. Soc. 68, 459 ; Oncley et al, 1949, J. Am. Chem. Soc. 71, 541). Les indications thérapeutiques des immunoglobulines s'étant multipliées, il existe un besoin de produit toujours plus performant et plus pur.

La complexité de la structure des immunoglobulines (quatre chaînes polypeptidiques réunies par des ponts disulfure), la variété des anticorps présents dans le mélange du plasma de plusieurs milliers de donneurs ne sont pas actuellement des facteurs favorisant le développement biotechnologique des immunoglobulines. Bien que des anticorps monoclonaux soient produits par ingénierie génétique, leur extrême spécificité constitue un inconvénient pour les applications thérapeutiques où une polyréactivité semble nécessaire.

De plus, de nombreuses pathologies, en particulier d'orgine autoimmune, sont actuellement traitées par des concentrés d'IgG. Cette large utilisation a engendré une situation de pénurie en Europe et aux Etats-Unis, au cours des dernières années.

Par ailleurs, dans ces mêmes pathologies, l'efficacité de préparations enrichies en IgM a été récemment démontrée. (Hurez et al. Blood 90, 1997, 4004-4013), mais il n'existe pas de préparation à usage thérapeutique suffisamment purifiée et concentrée en IgM.

C'est pourquoi la Demanderesse s'est attachée à la mise au point d'un nouveau procédé de préparation d'immunoglobulines humaines. Le procédé peut s'appliquer à un "pool" de sérums (d'au moins 10000 donneurs) ce qui assure la présence de tous les anticorps normalement présents dans l'ensemble de la population d'une contrée choisie, ou à des sérums hyperimmuns sélectionnés pour leur contenu en immunoglobulines spécifiques. Le procédé permet en outre de préparer des concentrés d'IgA et d'IgM.

De nombreuses variantes du procédé original de Cohn ont été décrites. Elles proposent, outre la précipitation sélective des protéines à l'éthanol, divers traitements additionnels comme la précipitation au polyéthylène glycol, le traitement ménagé aux enzymes protéolytiques..., destinés à éliminer les agrégats de polymères d'inununoglobulines (susceptibles d'activer le système du complément et de provoquer des réactions anaphylactiques).

Une voie alternative à la précipitation par l'éthanol a été décrite par Steinbuch et al. (Rev. Franç. Et. Clin. et Biol. 1969, XIV, 1054) qui fait appel à une précipitation par l'acide octanoïque. Celui-ci précipite la plupart des protéines du plasma et laisse dans le surnageant les immunoglobulines. La purification de ces immunoglobulines est poursuivie par adsorption (en "batch") sur un échangeur d'anions, la DEAE-cellulose, qui laisse également les immunoglobulines dans le surnageant. Celui-ci est ensuite concentré.

Divers procédés ont également été mis au point pour augmenter la pureté des produits par la mise en oeuvre de séparations chromatographiques. Les plus performants (notamment EP 0 703 922, WO 99/64462) comprennent au moins deux étapes successives de chromatographie, l'une par. échange d'anions, l'autre par échange de cations. La spécificité de ces procédés est apportée par la propriété des échangeurs d'anions de ne pas retenir les immunoglobulines G, dans les conditions classiques de mise en oeuvre, mais de fixer la plupart des autres protéines co-purifiées au cours des étapes de prépurification.

Diverses préparations d'IgG purifiées sont donc déjà disponibles mais leurs procédés de préparation posent encore des problèmes de rendement et de lourdeur de mise en oeuvre à l'échelle industrielle. Ces problèmes sont encore amplifiés par la nécessité d'inclure dans le procédé une inactivation virale impliquant une étape additionnelle d'élimination des agents virucides utilisés.

De plus, l'ensemble des procédés actuellement décrits ont été développés et optimisés pour la production des seules IgG.

La Demanderesse a trouvé qu'il était possible de produire plusieurs concentrés d'immunoglobulines en combinant une étape de prépurification et une étape unique de chromatographie par échange d'ions et ainsi de mettre en oeuvre un procédé très simple, à haut rendement et compatible avec un traitement virucide au solvant/détergent.

Ainsi le procédé selon la présente invention est applicable à du plasma sanguin ou à une fraction de plasma sanguin déjà enrichie en immunoglobulines et il comprend une prépurification par précipitation de contaminants lipidiques et protéiques et une chromatographie unique sur échangeur d'azzions, effectuée à pH alcalin ce qui permet l'adsorption des immunoglobulines sur le support chromatographique échangeur d'anions.

La prépurification est effectuée au moyen d'agents précipitants connus tels que l'acide octanoique, le phosphate tricalcique, la bentonite.

Après cette étape de prépurification et avant la chromatographie, le procédé comprend un traitement d'inactivation virale, de préférence par solvant-détergent, selon une méthode connue.

A la fin de ce traitement, le mélange du filtrat prépurifié et du solvant-déterqent est soumis à une séparation chromatographique qui est effectuée sur un gel de polysaccharide réticulé ou de polymère vinylique, greffé de groupements DEAE, TMAE ou QAE. Cette étape de chromatographie comprend
- l'ajustement à un pH de 8,9 à 9,1 de la solution ayant subi le traitement au solvant-détergent ;
- sa charge sur la colonne de chromatographie préalablement équilibrée en tampon à pH 8,9 à 9,1 ce qui permet l'adsorption des immunoglobulines et le passage des protéines non adsorbées dans l'effluent ;
- un lavage par le même tampon jusqu'à élimination de toutes les protéines non adsorbées et du mélange solvant-détergent ;
- et l'élution des immunoglobulines par un tampon approprié.

Cette élution peut être réalisée soit par du tampon phosphate à pH compris entre 4 et 7, et de préférence à pH 6,2 pour éluer les immunoglobulines G, soit de manière séquentielle :
- dans un premier temps comme ci-dessus pour éluer les immunoglobulines G ;
- dans un deuxième temps, par le même tampon phosphate additionné de NaCl 100 à 175 mM, et de préférence 150 mM, à un pH de 6 à 6,3, pour éluer une fraction contenant les IgA et des IgG4.

Le procédé peut être encore poursuivi par une nouvelle élution par le même tampon ajusté à un pH de 6 à 7 et additionné de NaCl 250 à 350 mM, de préférence de 300 mM pour éluer les IgM.

Les immunoglobulines ainsi éluées sont récoltées, concentrées par ultrafiltration et soumises à une filtration stérilisante conventionnelle puis à une filtration au travers de filtres nanométriques de porosité décroissante de 100 à 15 nanomètres, en particulier sur trois filtres disposés en série et de seuils de rétention décroissants, de 100, 50 et 20 nanomètres. Cette nanofiltration permet d'éliminer des virus résistants au traitement par solvant-détergent.

La solution d'immunoglobulines concentrée et filtrée est additionnée d'un stabilisant pharmaceutiquement acceptable, puis elle est conditionnée à l'état de solution stérile et éventuellement congelée et lyophilisée.

Les exemples suivants illustrent l'invention sans toutefois en limiter la portée.

### Exemple I

On utilise comme matériau de départ 1 Kg de précipité "I+II+III" obtenu à partir de plasma traité à l'éthanol selon la méthode de Cohn (déjà cité) ou de Kistler et Nitschmann (1962, Vox Sang. 7, 414).

Ce précipité est remis en suspension en tampon acétate (acétate de sodium-acide acétique) à pH 4,7-4,9, sous agitation, à 20°C.

On y ajoute de l'acide octanoïque jusqu'à une concentration finale de 20 g/l. L'addition doit être effectuée lentement, à température ambiante.

Le mélange est additionné d'un adjuvant de filtration et le précipité est séparé par filtration au moyen d'un filtre-presse.

Le filtrat est récupéré, clarifié et concentré par ultrafiltration, puis il est soumis à une filtration stérilisante à 0,45 µm et 0,2 µm.

Il est ensuite soumis à un traitement d'inactivation virale par solvant/détergent comme décrit par Neurath et Horowitz (brevet US 4,764,369). On utilise un mélange Triton® X 100 (octoxinol 10)/TnBP.

Le mélange est ajusté à 64 g/l de protéines, à pH 6,5. Le contact est maintenu pendant 4 à 6 heures, entre 4 et 25°C. Ensuite le pH est ajusté à 9 (par NaOH).

Le mélange est ensuite soumis à une chromatographie d'échange d'anions sur colonne.

On utilise comme matériau échangeur d'anions du TMAE-Fractogel®, chargé dans une colonne de chromatographie, équilibré en tampon glycine-NaCl (glycine 0,676 g/l - NaCl 0,526 g/l) à pH 9.

Le mélange est chargé sur la colonne à raison de 50 g de protéines pour 1 litre de gel.

Après la charge, la colonne est lavée en tampon glycine - NaCl, pH 9 (le même que pour l'équilibrage). Le lavage est surveillé par la densité optique de l'effluent à 280 nm.

Après retour à la ligne de base, la colonne est éluée avec du tampon phosphate à pH 6,2 (hydrogénophosphate disodique - dihydrogénophosphate de sodium).

L'éluat, contenant les immunoglobulines G, est ajusté à pH 4,5 et soumis à une ultrafiltration sur cassettes.

La solution est ensuite soumise à une filtration stérilisante à 0,22 µm, puis à une filtration nanométrique sur trois filtres disposés en série et de seuils de rétention décroissants, de 100, 50 et 20 nanomètres. La filtration est suivie d'une ultrafiltration sur cassette pour concentrer la solution finale à 120-150 g/l.

Les résultats d'analyse de trois lots pilotes sont présentés dans le tableau suivant.

**Tableau I. :**

| LOTS PILOTES | | | |
|---|---|---|---|
| | 00 ILP 043 PV | 00 ILP 044 PV | 00 ILP 045 PV |
| Polymères (%) | 0.19 | 0.19 | 0.07 |
| Dimères (%) | 3.29 | 2.27 | 2.15 |
| Monomères (%) | 96.52 | 97.54 | 97.78 |
| IgG (g/l) | 52.5 | 50.4 | 50.8 |
| IgG1 (g/l) | 31.6 | 29.9 | 29.2 |
| IgG2 (g/l) | 17.1 | 15.3 | 13.1 |
| IgG3 (g/l) | 1.19 | 1.07 | 0.96 |
| IgG4 (g/l) | 0.52 | 0.61 | 0.56 |
| IgM (µg/l) | 310 | 459 | 1795 |
| Anti-Hbs (UI/ml) | 10.2 | 10.3 | 9.4 |

### Exemple II

On utilise comme matériau de départ 1670 g de "précipité II" obtenu à partir de 80 litres de plasma traité à l'éthanol selon la méthode de Cohn.

Cette variante du procédé permet notamment de tirer parti de précipités II congelés et stockés en attente de traitement.

Ce précipité est remis en suspension dans 9 kg d'eau-NaCl. Le pH est ajusté à 6,2 par de l'acide acétique.

On y ajoute, comme adsorbant (des contaminants de type lipidiques, kallikréine...), 15 g de phosphate tricalcique.

Après une heure de contact, le mélange est additionné d'un adjuvant de filtration et le précipité est séparé par filtration au moyen d'un filtre-presse.

Le filtrat est récupéré, est additionné de glycine (15 g/l) et ajusté à pH 4,8 par de l'acide acétique.

On y ajoute, comme adsorbant (notamment des traces de facteurs de coagulation activés) 80 g de bentonite, sous agitation pendant 30 minutes.

Le mélange est additionné d'un adjuvant de filtration et le précipité est séparé par filtration au moyen d'un filtre-presse.

Le filtrat est récupéré, concentré par ultrafiltration.

Le procédé est poursuivi (par traitement au solvant/détergent et chromatographie) comme dans l'exemple I.

Les résultats d'analyse de trois lots pilotes sont présentés dans le tableau suivant.

**Tableau II :**

| LOTS PILOTES. | | | |
|---|---|---|---|
| | 286 | 287 | 321 |
| Polymères (%) | 0,5 | 0,5 | < 0,1 |
| Dimères (%) | 2,8 | 2,8 | 1,4 |
| Monomères (%) | 96,7 | 96,7 | 98,5 |
| IgG (g/l) | 51,8 | 53,2 | 61,3 |
| IgG1 (%) | 62,5 | 62,6 | 64,4 |
| IgG2 (%) | 33,0 | 33,0 | 32,2 |
| IgG3 (%) | 3,2 | 3,3 | 2,2 |
| IgG4 (%) | 1,2 | 1,2 | 0,8 |
| IgA (mg/l) | 26,4 | 21,5 | 12,6 |
| IgM (µg/l) | 90,8 | 69,4 | 123 |
| anti-Hbs (UI/ml) | 147 | 122 | 8,6 |

### Exemple III

Le procédé est mis en oeuvre de la même manière que dans l'exemple I mais le matériau de départ est du plasma (non précipité à l'éthanol).

Cette variante n'est pas avantageuse au point de vue du rendement mais elle présente un intérêt (par son nombre réduit d'étapes) pour le traitement de plasmas particulièrement riches en anticorps rares.

### Exemple IV

Le procédé est mis en oeuvre de la même manière que dans l'exemple I mais la chromatographie est éluée de manière séquentielle :
après la charge et le lavage de la colonne, celle-ci est éluée avec un premier tampon phosphate à pH 6,2 pour éluer les IgG comme dant l'exemple I ;
ensuite elle est éluée avec le même tampon additionné de NaCl 150 mM, qui élue une fraction enrichie en IgA et en IgG4 ;
Les IgG4 semblent jouer un rôle dans la protection contre les infections parasitaires et contre les allergies aux pollens et aux acariens.

De manière surprenante leurs propriétés biochimiques et leur comportement en échange d'ions les apparentent aux IgA.

### Exemple V

Le procédé est mis en oeuvre de la même manière que dans l'exemple IV mais après la deuxième élution qui produit la fraction enrichie en IgA, le même tampon est additionné de NaCl 300 mM ce qui décroche les IgM. on obtient ainsi une fraction concentrée (à 80 %) d'IgM. Celles-ci sont concentrées et additionnées de stabilisants pharmaceutiquement accepables.

Les résultats de production d'un lot d'IgM concentrées sont présentés dans le tableau suivant.

**Tableau III**

| (Lot IgM 2000-97) | | | | | | |
|---|---|---|---|---|---|---|
| Fraction | Volume (ml) | Ig Totales (mg) | IgM (mg) | IgG (%) | IgA (%) | IgM (%) |
| Ig prépurifiées | 2190 | 39957 | 2037 | 92.6 | 2,2 | 5.1 |
| Fraction non retenue sur la colonne | 3800 | 566,8 | <32.3 | 86.5 | <7.8 | <5.7 |
| Eluat 1 (IgG) | 2700 | 37374 | <22.9 | 99.8 | <0.1 | <0.1 |
| Eluat 2 (IgA) (0.15M NaCl) | 1350 | 3171 | 62.5 | 74.9 | 23.1 | 2 |
| Eluat 3 (IgM) (0.3M NaCl) | 114.5 | 1775.1 | 1545.7 | 6.2 | 6.8 | 87.1 |

## Revendications

1. Procédé de préparation de concentrés d'immunoglobulines humaines à usage thérapeutique à partir de plasma sanguin ou d'une fraction de plasma enrichie en immunoglobulines, **caractérisé en ce qu'**il comprend une prépurification par précipitation de contaminants lipidiques et protéiques et une unique étape de chromatographie, cette étape de chromatographie étant effectuée sur un échangeur d'anions à pH alcalin ce qui permet l'adsorption des immunoglobulines sur ledit échangeur d'anions.

2. Procédé selon la revendication 1, **caractérisé en ce que** la prépurification est effectuée au moyen d'agents précipitants connus, tels que l'acide octanoïque, le phosphate tricalcique, la bentonite.

3. Procédé selon la revendication 1 ou 2 **caractérisé en ce qu'**il comporte, après la prépurification et avant la chromatographie, un traitement d'inactivation virale, de préférence par solvant-détergent.

4. Procédé selon l'une quelconque des revendications 1 à 3, **caractérisé en ce que** la chromatographie est effectuée sur un gel de polysaccharide réticulé ou de polymère vinylique, greffé de groupements DEAE ou TMAE ou QAE.

5. Procédé selon l'une quelconque des revendications 1 à 4, **caractérisé en ce que** la chromatographie comprend
- l'utilisation de la solution ayant subi le traitement au solvant-détergent ;
- son ajustement à un pH de 8,9 à 9,1 ;
- sa charge sur la colonne de chromatographie préalablement équilibrée en tampon à pH 8,9 à 9,1 ce qui permet l'adsorption des immunoglobulines et le passage des protéines non adsorbées dans l'effluent ;
- un lavage par le même tampon jusqu'à élimination de toutes les protéines non adsorbées et du mélange solvant-détergent ;
- et l'élution des immunoglobulines par un tampon approprié.

6. Procédé selon la revendication 5, **caractérisé en ce que** l'élution est réalisée en une étape, par du tampon phosphate à pH compris entre 4 et 7, et de préférence à pH 6,2, pour éluer les immunoglobulines G.

7. Procédé selon la revendication 5, **caractérisé en ce que** l'élution est réalisée de manière séquentielle,
- dans un premier temps, par du tampon phosphate à pH compris entre 4 et 7, et de préférence à pH 6,2, pour éluer les immmunoglobulines G ;
- dans un deuxième temps, par le même tampon phosphate additionné de NaCl 100 à 175 mM, et de préférence 150 mM, à un pH de 6 à 6,3, pour éluer une fraction contenant les IgA et des IgG4 .

8. Procédé selon la revendication 6 ou 7, **caractérisé en ce qu'**il comprend, en outre, une élution par le même tampon ajusté à un pH de 6 à 7 et additionné de NaCl 250 à 350 mM, de préférence 300 mM pour éluer les IgM.

9. Procédé selon l'une quelconque des revendications 1 à 8, **caractérisé en ce que**, après l'élution des immunoglobulines, celles-ci sont récoltées, concentrées par ultrafiltration et soumises à une filtration stérilisante conventionnelle puis à une filtration au travers de filtres nanométriques de porosité décroissante de 100 à 15 nanomètres.

10. Procédé selon l'une quelconque des revendications 1 à 9, **caractérisé en ce que** la solution d'immunoglobulines concentrée et filtrée est additionnée d'un stabilisant pharmaceutiquement acceptable puis elle est conditionnée à l'état de solution stérile et éventuellement congelée et lyophilisée.

## Patentansprüche

1. Verfahren zur Herstellung von Human-Immunglobulin-Konzentraten für die therapeutische Verwendung aus Blutplasma oder einer Plasmafraktion, die an Immunglobulinen angereichert ist,
**dadurch gekennzeichnet, dass** das Verfahren umfasst
eine Vorreinigung durch Präzipitation von Lipid- und Protetn-Kontaminanten und eine einzige Chromatographie-Stufe, die unter Verwendung eines AnionenAustauschers bei einem alkalischen pH-Wert durchgeführt wird, wodurch die Adsorption der Immunglobuline an dem Anionen-Austauscher ermöglicht wird.

2. Verfahren nach Anspruch 1, **dadurch gekennzeichnet, dass** die Vorreinigung mittels bekannter Präzipitationsmittel, wie z.B. Octansäure, Tricalciumphosphat, Bentonit, durchgeführt wird.

3. Verfahren nach Anspruch 1 oder 2, **dadurch gekennzeichnet dass** es nach der Vorreinigung und vor der Chromatographie eine Virus-Inaktivierungsbehandlung, vorzugsweise mit einem Lösungsmittel-Detergens, umfasst.

4. Verfahren nach einem der Ansprüche 1 bis 3, **dadurch gekennzeichnet, dass** die Chromatographie an einem vernetzten Polysaccharid- oder Vinylpolymer-Gel mit aufgepfropften DEAE- oder TMAE- oder QAE-Gruppen durchgeführt wird.

5. Verfahren nach einem der Ansprüche 1 bis 4, **dadurch gekennzeichnet, dass** die Chromatographie umfasst
- die Verwendung einer Lösung, die einer Lösungsmittel-Detergens-Behandlung unterworfen worden ist;
- ihre Einstellung auf einen pH-Wert von 8,9 bis 9,1;
- ihre Aufgabe auf die vorher in einem Puffer bei pH 8,9 bis 9,1 äquilibrierte Chromatographie-Kolonne, welche die Adsorption der Immunglobuline und den Durchlauf der nicht adsorbierten Proteine in den Abstrom ermöglicht;
- das Waschen mit dem gleichen Puffer bis zur Eliminierung aller nichtadsorbierten Proteine und der Lösungsmittel-Detergens-Mischung; und
- das Eluieren der Immunglobuline mit einem geeigneten Puffer.

6. Verfahren nach Anspruch 5, **dadurch gekennzeichnet, dass** das Eluieren in einer Stufe mit einem Phosphat-Puffer bei einem pH-Wert zwischen 4 und 7 und vorzugsweise bei pH 6,2 durchgeführt wird, um die Immunglobuline G zu eluieren.

7. Verfahren nach Anspruch 5, **dadurch gekennzeichnet, dass** das Eluieren sequentiell durchgeführt wird,
- in einer ersten Stufe mit einem Phosphat-Puffer bei einem pH-Wert zwischen 4 und 7 und vorzugsweise bei pH 6,2, um die Immunglobuline G zu eluieren; und
- in einer zweiten Stufe mit dem gleichen Phosphat-Puffer, dem 100 bis 175 mM NaCl, vorzugsweise 150 mM NaCl zugesetzt worden sind, bei einem pH-Wert von 6 bis 6,3, um eine Fraktion zu eluieren, welche die IgA und IgG4 enthält.

8. Verfahren nach Anspruch 6 oder 7, **dadurch gekennzeichnet, dass** es außerdem umfasst ein Eluieren mit dem gleichen Puffer, der auf einen pH-Wert von 6 bis 7 eingestellt worden ist und dem 250 bis 350 mM NaCl, vorzugsweise 300 mM NaCl zugesetzt worden sind, um die IgM zu eluieren.

9. Verfahren nach einem der Ansprüche 1 bis 8, **dadurch gekennzeichnet, dass** nach dem Eluieren der Immunglobuline diese abgetrennt (gewonnen), durch Ultrafiltration konzentriert und einer konventioneller: sterilisierenden Filtration und danach einer Filtration durch Nanometer-Filter mit einer von 100 nm auf 15 nm abnehmenden Porosität unterworfen werden.

10. Verfahren nach einem der Ansprüche 1 bis 9, **dadurch gekennzeichnet, dass** die konzentrierte und filtrierte Immunglobulin-Lösung mit einem pharmazeutisch akzeptablen Stabilisierungsmittel versetzt und dann im Zustand einer sterilen Lösung konditioniert und gegebenenfalls eingefroren und lyophilisiert wird.

## Claims

1. A method for preparing human immunoglobulin concentrates for therapeutic use from blood plasma or from a plasma fraction enriched in immunoglobulins, **characterized in that** it comprises a prepurification by precipitation of lipid and protein contaminants and a single chromatography step, this chromatography step being carried out on an anion exchanger at an alkaline pH, which allows the immunoglobulins to be adsorbed onto said anion exchanger.

2. A method according to claim 1, **characterized in that** the prepurification is carried out by means of known precipitating agents, such as octanoic acid, tricalcium phosphate or bentonite.

3. A method according to claim 1 or 2, **characterized in that** it comprises, after the prepurification and before the chromatography, a viral inactivation treatment, preferably with solvent-detergent.

4. A method according to any one of claims 1 to 3, **characterized in that** the chromatography is carried out on a gel of crosslinked polysaccharide or of vinyl polymer, grafted with DEAE or TMAE or QAE groups.

5. A method according to any one of claims 1 to 4, **characterized in that** the chromatography comprises
- use of the solution that has undergone the solvent-detergent treatment;
- adjustment thereof to a pH of from 8.9 to 9.1;
- loading thereof onto the chromatography column preequilibrated in buffer at from pH 8.9 to 9.1, which allows the immunoglobulins to be adsorbed and the non-adsorbed protein to pass into the effluent;
- washing with the same buffer until all the non-adsorbed proteins and the solvent-detergent mixture have been eliminated;
- and elution of the immunoglobulins with an appropriate buffer.

6. A method according to claim 5, **characterized in that** the elution is carried out in one step, with phosphate buffer at a pH of from 4 to 7, and preferably at 6.2, in order to elute the immunoglobulins G.

7. A method according to claim 5, **characterized in that** the elution is carried out sequentially:
- firstly, with phosphate buffer at a pH of from 4 to 7, and preferably at a pH of 6.2, in order to elute the immunoglobulins G;
- secondly, with the same phosphate buffer to which from 100 to 155 mM, and preferably 150 mM, NaCl have been added, at a pH of from 6 to 6.3, in order to elute a fraction containing the IgAs and IgG4s.

8. A method according to claim 6 or 7, **characterized in that** it also comprises an elution with the same buffer, adjusted to a pH of from 6 to 7 and to which from 250 to 350 mM, preferably 300 mM, NaCl has been added, in order to elute the IgMs.

9. A method according to any one of claims 1 to 8, **characterized in that**, after the elution of the immunoglobulins, the latter are harvested, concentrated by ultrafiltration and subjected to conventional sterilizing filtration and then to filtration through nanometric filters having a decreasing porosity of from 100 to 15 nanometres.

10. A method according to any one of claims 1 to 9, **characterized in that** a pharmaceutically acceptable stabilizer is added to the concentrated and filtered immunoglobulin solution, which is then packaged in the form of a sterile solution and, optionally, frozen and lyophilized.
